# EUROPEAN PATENT SPECIFICATION

(11) **EP 4 138 691 B1**
(45) Date of publication and mention of the grant of the patent: **18.06.2025**
(21) Application number: 21793062.7
(22) Date of filing: 15.04.2021
(51) Int. Cl.: A61B 17/34, A61M 25/06, A61M 29/00, A61B 17/00

(54) **SHEATH ASSEMBLY AND/OR DILATOR ASSEMBLY**
HÜLLENANORDNUNG UND/ODER DILATATORANORDNUNG
ENSEMBLE GAINE ET/OU ENSEMBLE DILATATEUR

(30) Priority: 22.04.2020 US 202063013646 P
(43) Date of publication of application: 01.03.2023
(73) Proprietor: Boston Scientific Medical Device Limited, Galway H91 Y868 (IE)
(72) Inventor: LEUNG, Jackie, Mississauga, Ontario L4W 5P6 (CA); DAVIES, Gareth, Mississauga, Ontario L4W 5P6 (CA)
(74) Representative: Pfenning, Meinig & Partner mbB
(86) International application number: PCT/IB2021/053119
(87) International publication number: WO 2021/214607

(56) References cited:
- WO-A1-2019/215618
- WO-A1-2019/215623
- JP-A- 2018 519 957
- US-A- 4 907 599
- US-A1- 2009 043 302
- US-A1- 2009 043 302
- US-A1- 2015 359 539
- US-A1- 2015 359 539
- US-A1- 2020 107 823
- US-A1- 2020 107 823

## Description

### TECHNICAL FIELD

This document relates to the technical fields of (and is not limited to) (A) a kit including an elongated sheath assembly, an elongated dilator assembly, a dilator-receiver device and a dilator-receiver mover; and (B) an apparatus for use with (configured to be used with) an elongated dilator assembly, in which the apparatus includes an elongated sheath assembly (configured to receive the elongated dilator assembly), a dilator-receiver device and a dilator-receiver mover; and (C) an apparatus for installation to an elongated sheath assembly (configured to receive an elongated dilator assembly), in which the apparatus includes a dilator-receiver device and a dilator-receiver mover; and (D) a method for operating an elongated sheath assembly configured to receive an elongated dilator assembly; and (E) a kit including a first medical assembly, a second medical assembly, a receiver device, and a receiver mover, etc.

### BACKGROUND

Known medical devices are configured to facilitate a medical procedure, and help healthcare providers diagnose and/or treat medical conditions.

Document US 2020-0107823 A1 discloses an apparatus for sealing a puncture through a vessel wall including a positioning assembly, a sheath releasably engaged with the positioning assembly, and a support member axially advanceable through the sheath. The positioning assembly includes a positioning element positioned at a distal portion of the positioning assembly and a sealant disposed at a distal portion of the positioning assembly. The sheath guides the sealant and positioning assembly to the puncture in the vessel wall.

Document US 2009-0043302 A1 discloses systems and methods to deploy an electrode from a catheter assembly. The systems and methods provide a catheter handle having a trigger lever adapted to carry an actuator rod. The actuator rod is adapted to cause movement of the electrode between a retracted position and an extended position. A pinion is carried by the trigger lever for engagement with a rack carried by the actuator rod. Compression of the trigger lever moves the rack along the actuator rod between a first position corresponding to the electrodes being in the retracted position and a second position corresponding to either the primed electrode firing position or the electrodes being in an extended position.

Document US 2015-0359539 A1 discloses a system for delivery and deployment of an occluder including a handle, an actuating mechanism with a motion amplifier and a sheath positioning mechanism within the handle. A driveshaft of the actuating mechanism extends through the handle, the sheath positioning mechanism, and an attached sheath so as to couple with an occluder deployed via the driveshaft within a body lumen in a patient.

Document US 4907599 A discloses a soft tissue core biopsy instrument comprising a proximal outer barrel member and a distal inner barrel member which is slidably received within the outer barrel member. A cannula driver is slidably received within the distal inner barrel member and has a distally extending cannula fixed thereto. A stylet extends distally from a stylet hub with a stylet hub being removably attached to the proximal outer barrel member. A spring is positioned within the inner barrel member between a proximal wall thereof and a proximal wall of the cannula driver for biasing the cannula driver forwardly and a latching mechanism for latching the cannula driver in a proximally retracted position against the action of spring is also provided. A trigger for unlatching the latching mechanism may be actuated to allow the spring to quickly, return to its forwardly biased position, shooting or forcing the cannula driver to a distally extended position, projecting the cannula over said stylet.

Document JP 2018-519957 A appears to disclose a multi-function instrument platform is that can be variably configured by clinicians to treat occlusions. The Functionally Integrable Catheter System refers to a system of "functional units" that can be configured together to operate synergistically, and includes at least four main "functional units" including: (a) a Functionally Integrable Catheter System support catheter (110), (b) a Functionally Integrable Catheter System dilator (130), (c) a Functionally Integrable Catheter System PTA catheter (140), and (d) a Functionally Integrable Catheter System lock-grip handle (120). Each "functional unit" may be provided in a pre-assembled form by the manufacturer, intended to be configured into variable combinations by the clinical worker.

Document WO 2019-215618 A1 discloses methods and devices for puncturing tissue, comprising a puncture device for puncturing tissue and a supporting member for supporting the puncture device. The puncture device is capable of being insertable within the supporting member and being selectively usable in co-operation therewith during a portion of a procedure for puncturing tissue and wherein the puncture device is usable independently therefrom during another portion of the procedure. The puncture device comprises visual or tactile markers for determining the relative positioning between puncture device and supporting member.

Document WO 2019-215623 A1 discloses apparatus and systems that incorporate coupling mechanisms to enable coupling of two mating member such as medical devices such as introducers, sheaths, dilators. More specifically, the disclosure relates to releasable coupling mechanisms, to allow for releasable coupling of two medical devices such as a dilator and a sheath so the devices can be maneuvered and/or manipulated together for example during a part of a medical procedure.

### SUMMARY

It will be appreciated that there exists a need to mitigate (at least in part) at least one problem associated with the existing (known) sheath assemblies (also called the existing technology). After much study of, and experimentation with, the existing (known) sheath assemblies, an understanding (at least in part) of the problem and its solution have been identified (at least in part) and are articulated (at least in part) as follows:

Transseptal puncture of the fossa ovalis (of the heart of a patient) may require a puncture device to first be positioned by a guiding device (such as a sheath/dilator combination). The sheath/dilator combination is configured to push against, and tent, the septum to provide visual confirmation through or via a medical imaging system. The issue lies in controlling the pressure that may be applied onto the septum. Insufficient force may not result in visible tenting, while excessive force may increase the risk of the sheath/dilator springing forward into the left atrium after puncture.

FIG. 1A and FIG. 2B depicts a side perspective view of an embodiment of a known elongated sheath assembly, and an elongated dilator assembly for use with, or configured to be used with (to be inserted, at least in part) into the known elongated sheath assembly.

FIG. 2A and FIG. 2B depicts a side perspective view (FIG. 2A) and a side view (FIG. 2B) of embodiments of the known elongated dilator assembly of FIG. 1B inserted into the elongated sheath assembly of FIG. 1A.

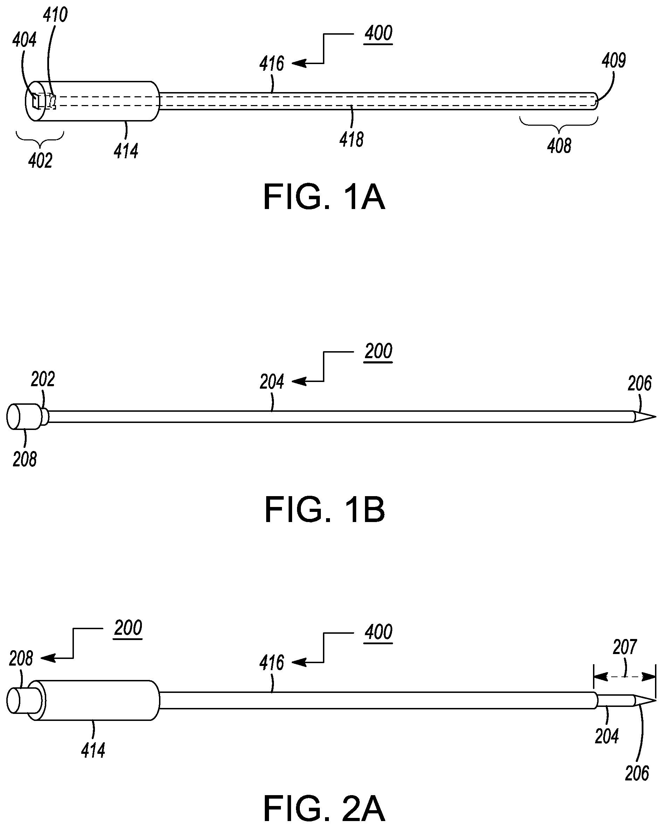

Referring to the embodiment as depicted in FIG. 1A, the known sheath assembly includes a first end section defining an entrance portal. A second end section is spaced apart from the first end section. The second end section defines an exit portal. A stationary shaft extends between the first end section and the second end section. The stationary shaft defines a shaft lumen extending along a longitudinal length of the sheath assembly. The stationary shaft has a fixed length (a predetermined length). The entrance portal and the exit portal are in fluid communication with each other via the shaft lumen. A sheath-handle assembly is positioned at the first end section. A sheath hub is positioned at the first end section. Preferably, the sheath hub is positioned in the interior of the sheath-handle assembly.

Referring to the embodiment as depicted in FIG. 1B, the elongated dilator assembly includes a dilator handle at one end section (of the elongated dilator assembly), and a dilator distal tip positioned at the opposite end section (of the elongated dilator assembly). A dilator hub is positioned at the dilator handle, and faces or is oriented toward the dilator distal tip. An elongated dilator shaft extends between the dilator hub and the dilator distal tip. The dilator assembly has a fixed length (a predetermined length).

Referring to the embodiment as depicted in FIG. 2A, the sheath assembly is mated with the elongated dilator assembly. The sheath assembly is configured to receive (slidably receive) the elongated dilator assembly within the shaft lumen of the stationary shaft (along the longitudinal length of the sheath assembly). Once the sheath assembly, in use, fully receives (is fully mated with) the elongated dilator assembly within the shaft lumen, the front section of the elongated dilator assembly extends outwardly from the front section (of the sheath assembly) to expose (display) a fixed exposed dilator shaft portion having a fixed length (a predetermined shaft length, which is a fixed portion of the dilator shaft).

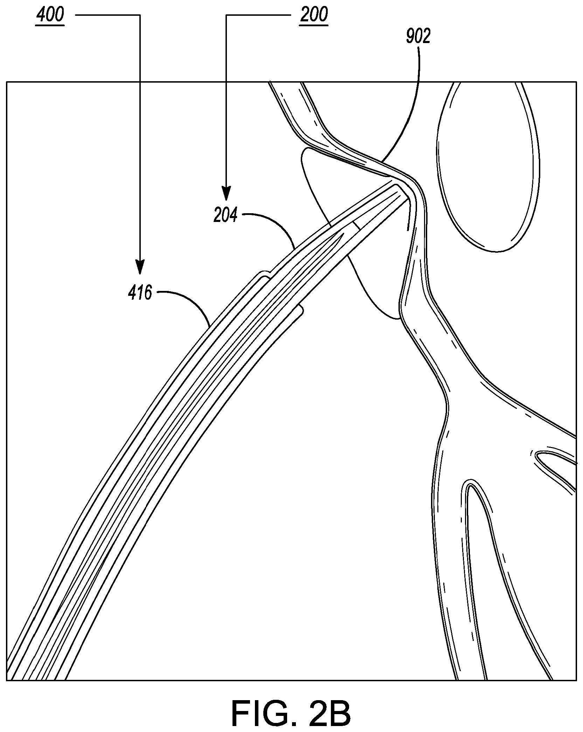

Referring to the embodiment as depicted in FIG. 2B, the problem with this arrangement is that for the case where the reach of the elongated dilator assembly is too long or too short resulting in excessive or insufficient tenting of the septum (located in the right atrium of the heart of the patient), the surgeon may need to adjust (the orientation of and/or the alignment of) the curve (shape) of the elongated dilator assembly either through manual manipulation and/or by utilizing a steering mechanism (known and not depicted), thereby wasting or increasing valuable or critical surgical time. The dilator and the known sheath may be typically long enough for the procedure. The reach of the dilator may be controlled by how it is curved in the right atrium. One solution may be to adjust the bend (curve) of the dilator; however, this may require (A) pulling the dilator out from the known sheath and manually bending the dilator (if the dilator is shapeable), and/or (B) using a steerable known sheath (known and not depicted) that the surgeon (operator) may then control the curve of the dilator. Both methods may be imprecise and also may be dependent on the surgeon's experience and comfort with the procedure. It may be desirable to provide a workflow that starts with a long reach and tenting (over-tenting) the septum, then withdrawing the dilator to an appropriate degree (or amount) of tent formed in a portion of the septum; it will be appreciated that this desirable methodology may be more intuitive than changing the curve of the dilator.

Referring to the embodiment as depicted in FIG. 2A, it may be desirable to avoid the process of adjusting the curve of the elongated dilator assembly and thereby avoid wasting valuable or critical surgical time.

Referring to the embodiment as depicted in FIG. 2A, it may be desirable to provide an arrangement in which the additional length of the elongated dilator assembly, which becomes exposed during a procedure, in which the length thereof may be adjusted by the operator while the sheath assembly is positioned, thereby saving valuable or critical surgical time (as a result of).

Referring to the embodiment as depicted in FIG. 2A, it may be desirable to provide an arrangement in which the elongated dilator assembly may be selectively extended (moved further out from) or retracted into (pulled into) (the output of) a sheath assembly, while the elongated dilator assembly remains mated to the sheath assembly during a procedure, so that the elongated dilator assembly may become selectively moved (and exposed at least in part) from the end portion of the sheath assembly (during the procedure). Selectively moved means that the elongated dilator assembly is movable in response to receiving an application of a movement force; the elongated dilator assembly does not move of its own accord without application of a movement force.

To mitigate, at least in part, at least one problem associated with the existing technology, there is provided (in accordance with a major aspect) a kit. The kit includes and is not limited to (comprises) a first medical assembly. A second medical assembly is configured to receive, at least in part, the first medical assembly. A receiver device is configured to be mounted, at least in part, to the second medical assembly. The receiver device is also configured to receive the first medical assembly. A receiver mover is configured to be operatively connected to the receiver device in such a way that the receiver mover selectively moves the receiver device along a predetermined distance.

To mitigate, at least in part, at least one problem associated with the existing technology, there is provided (in accordance with a major aspect) a kit. The kit includes, and is not limited to (comprises) a synergistic combination of elements. The kit includes, for instance, an elongated dilator assembly and an elongated sheath assembly configured to receive, at least in part, the elongated dilator assembly. The kit also includes a dilator-receiver device configured to be (having a structure configured to be) mounted to, at least in part, the elongated sheath assembly. The kit also includes a dilator-receiver mover configured to be operatively connected to, at least in part, the dilator-receiver device. Preferably, the dilator-receiver mover is configured to selectively move the dilator-receiver device along a predetermined distance. In accordance with a preferred embodiment, the dilator-receiver mover is configured to be mounted, at least in part, to the elongated sheath assembly.

To mitigate, at least in part, at least one problem associated with the existing technology, there is provided (in accordance with a major aspect) an apparatus. The apparatus is for use with (configured to be used with) an elongated dilator assembly. The apparatus includes and is not limited to (comprises) an elongated sheath assembly configured to receive, at least in part, the elongated dilator assembly. A dilator-receiver device is configured to be mounted, at least in part, to the elongated sheath assembly. A dilator-receiver mover is operatively connected to the dilator-receiver device. Preferably, the dilator-receiver mover is configured to selectively move the dilator-receiver device along a predetermined distance.

To mitigate, at least in part, at least one problem associated with the existing technology, there is provided (in accordance with a major aspect) an apparatus. The apparatus is usable with (is for) an elongated sheath assembly. The elongated sheath assembly is configured to receive, at least in part, an elongated dilator assembly. The apparatus includes and is not limited to (comprises) a synergistic combination of a dilator-receiver device and a dilator-receiver mover. The dilator-receiver device is configured to be mounted, at least in part, to the elongated sheath assembly. The dilator-receiver mover is configured to be operatively connected, at least in part, to the dilator-receiver device. In accordance with a preferred embodiment, the dilator-receiver mover is configured to be mounted, at least in part, to the elongated sheath assembly. Preferably, the dilator-receiver mover is configured to selectively move the dilator-receiver device along a predetermined distance.

To mitigate, at least in part, at least one problem associated with the existing technology, there is provided (in accordance with a major aspect) a method. The method is for operating an elongated sheath assembly configured to receive, at least in part, an elongated dilator assembly. The method includes and is not limited to (comprises) selectively receiving and supporting, at least in part, the elongated dilator assembly via a dilator-receiver device mounted to, at least in part, the elongated sheath assembly. The method also includes selectively moving, at least in part, the dilator-receiver device and the elongated dilator assembly along a predetermined distance, via a dilator-receiver mover operatively connected to the dilator-receiver device, after the elongated dilator assembly is received, at least in part, by the dilator-receiver device. In accordance with a preferred embodiment, the dilator-receiver mover is configured to be mounted, at least in part, to the elongated sheath assembly. Preferably, the dilator-receiver mover is configured to selectively move the dilator-receiver device along the predetermined distance.

Other aspects are identified in the claims. Other aspects and features of the non-limiting embodiments may now become apparent to those skilled in the art upon review of the following detailed description of the non-limiting embodiments with the accompanying drawings. This Summary is provided to introduce concepts in simplified form that are further described below in the Detailed Description. This Summary is not intended to identify potentially key features or possible essential features of the disclosed subject matter, and is not intended to describe each disclosed embodiment or every implementation of the disclosed subject matter. Many other novel advantages, features, and relationships will become apparent as this description proceeds. The figures and the description that follow more particularly exemplify illustrative embodiments.

### BRIEF DESCRIPTION OF THE DRAWINGS

The non-limiting embodiments may be more fully appreciated by reference to the following detailed description of the non-limiting embodiments when taken in conjunction with the accompanying drawings, in which:

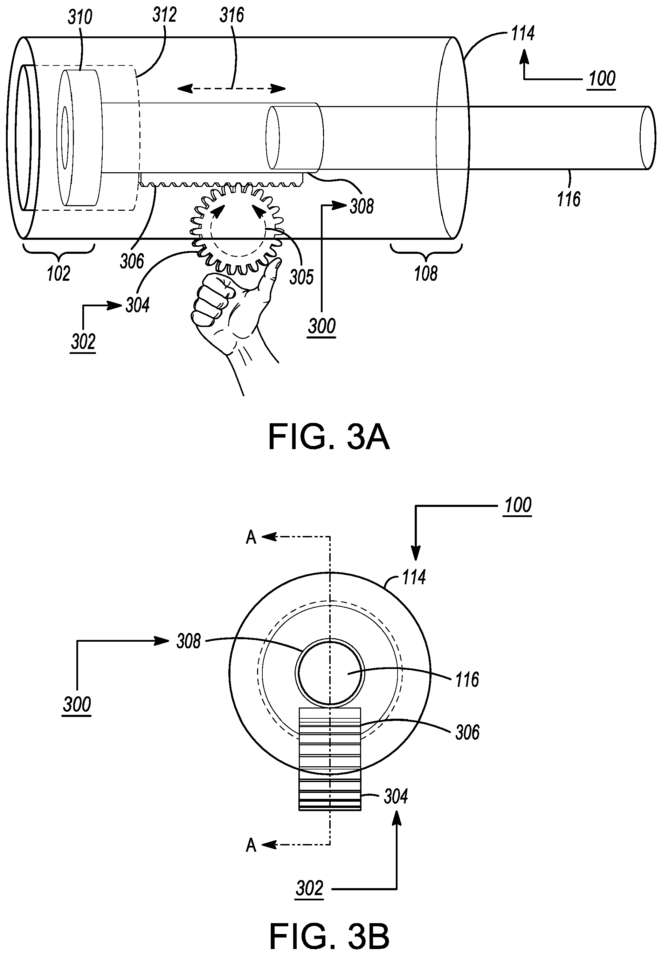

FIG. 3A and FIG. 3B depict a side perspective view (FIG. 3A) and a front end view (FIG. 3B) of embodiments of a dilator-receiver device and a dilator-receiver mover mounted to an elongated sheath assembly; and

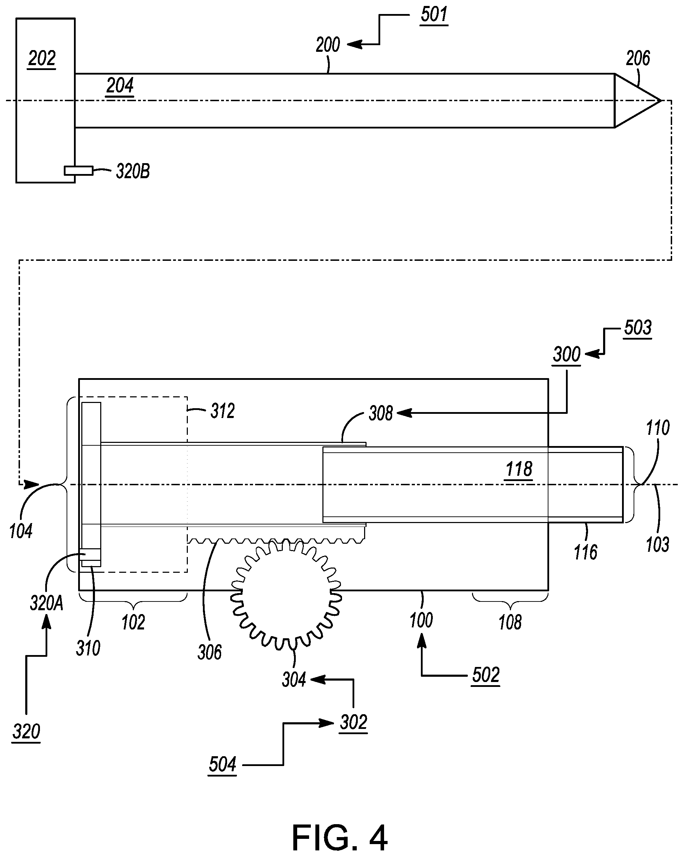

FIG. 4, FIG. 5, FIG. 6 and FIG. 7 depict cross-sectional views of the dilator-receiver device and the dilator-receiver mover mounted to the elongated sheath assembly of FIG. 3B.

The drawings are not necessarily to scale and may be illustrated by phantom lines, diagrammatic representations and fragmentary views. In certain instances, details unnecessary for an understanding of the embodiments (and/or details that render other details difficult to perceive) may have been omitted. Corresponding reference characters indicate corresponding components throughout the several figures of the drawings. Elements in the several figures are illustrated for simplicity and clarity and have not been drawn to scale. The dimensions of some of the elements in the figures may be emphasized relative to other elements for facilitating an understanding of the various disclosed embodiments. In addition, common, and well-understood, elements that are useful in commercially feasible embodiments are often not depicted to provide a less obstructed view of the embodiments of the present disclosure.

**LISTING OF REFERENCE NUMERALS USED IN THE DRAWINGS**

| | | | |
|---|---|---|---|
| sheath assembly | 100 | input gear | 304 |
| first end section | 102 | rotation direction | 305 |
| elongated axis | 103 | output gear | 306 |
| entrance portal | 104 | moveable shaft | 308 |
| second end section | 108 | sheath hub | 310 |
| exit portal | 110 | hub cavity | 312 |
| sheath-handle assembly | 114 | predetermined distance | 314 |
| stationary shaft | 116 | predetermined distance | 315 |
| shaft lumen | 118 | reciprocation path | 316 |
| dilator assembly | 200 | lock mechanism | 318 |
| insertion direction | 201 | lock device | 320 |
| dilator hub | 202 | first lock portion | 320A |
| dilator shaft | 204 | second lock portion | 320B |
| dilator distal tip | 206 | sheath assembly | 400 |
| dilator shaft portion | 207 | first end section | 402 |
| dilator handle | 208 | entrance portal | 404 |
| dilator-receiver device | 300 | second end section | 408 |
| dilator-receiver mover | 302 | exit portal | 409 |
| sheath hub | 410 | first medical assembly | 501 |
| sheath-handle assembly | 414 | second medical assembly | 502 |
| stationary shaft | 416 | receiver device | 503 |
| shaft lumen | 418 | receiver mover | 504 |

### DETAILED DESCRIPTION OF THE NON-LIMITING EMBODIMENT(S)

The following detailed description is merely exemplary and is not intended to limit the described embodiments or the application and uses of the described embodiments. As used, the word "exemplary" or "illustrative" means "serving as an example, instance, or illustration." Any implementation described as "exemplary" or "illustrative" is not necessarily to be construed as preferred or advantageous over other implementations. All of the implementations described below are exemplary implementations provided to enable persons skilled in the art to make or use the embodiments of the disclosure and are not intended to limit the scope of the disclosure. The scope of the disclosure is defined by the claims. For the description, the terms "upper," "lower," "left," "rear," "right," "front," "vertical," "horizontal," and derivatives thereof shall relate to the examples as oriented in the drawings. There is no intention to be bound by any expressed or implied theory in the preceding Technical Field, Background, Summary or the following detailed description. It is also to be understood that the devices and processes illustrated in the attached drawings, and described in the following specification, are exemplary embodiments (examples), aspects and/or concepts defined in the appended claims. Hence, dimensions and other physical characteristics relating to the embodiments disclosed are not to be considered as limiting, unless the claims expressly state otherwise. It is understood that the phrase "at least one" is equivalent to "a". The aspects (examples, alterations, modifications, options, variations, embodiments and/or any equivalent thereof) are described regarding the drawings. It should be understood that the disclosure is limited to the subject matter provided by the claims, and that the disclosure is not limited to the particular aspects depicted and described. It will be appreciated that the scope of the meaning of a device configured to be coupled to an item (that is, to be connected to, to interact with the item, etc.) is to be interpreted as the device being configured to be coupled to the item, either directly or indirectly. Therefore, "configured to" may include the meaning "either directly or indirectly" unless specifically stated otherwise.

FIG. 3A and FIG. 3B depict a side perspective view (FIG. 3A) and a front view (FIG. 3B) of embodiments of a dilator-receiver device 300 and a dilator-receiver mover 302 mounted to an elongated sheath assembly 100.

FIG. 4, FIG. 5, FIG. 6 and FIG. 7 depict cross-sectional views of the dilator-receiver device 300 and the dilator-receiver mover 302 mounted to the elongated sheath assembly 100 of FIG. 3B. An elongated dilator assembly 200 is for use with, or configured to be used with (to be inserted, at least in part, into) the elongated sheath assembly 100. The cross-sectional views of FIG. 4, FIG. 5, FIG. 6 and FIG. 7 are taken along the cross-sectional line A-A of FIG. 3B.

Referring to the embodiments as depicted in FIG. 3A, FIG. 3B and FIG. 4, there is depicted a kit. The kit includes an elongated dilator assembly 200 and an elongated sheath assembly 100 configured to receive, at least in part, the elongated dilator assembly 200. A dilator-receiver device 300 is configured to be mounted (installed), at least in part, to the elongated sheath assembly 100. The dilator-receiver device 300 includes (preferably) a moveable shaft 308 configured to be mounted, at least in part, to the elongated sheath assembly 100. The dilator-receiver device 300 has (preferably) a receiver structure configured to be mounted, at least in part, to the elongated sheath assembly 100. A dilator-receiver mover 302 is configured to be operatively connected (directly or indirectly, couple) to the dilator-receiver device 300; this is done in such a way that the dilator-receiver mover 302 selectively moves the dilator-receiver device 300 along a predetermined distance 314.

Referring to the embodiments as depicted in FIG. 3A, FIG. 3B and FIG. 4, the elongated dilator assembly 200 may include any type of sheath device, such as that type used for transseptal puncturing. The elongated dilator assembly 200 may be of a different size, shape and/or material. The elongated dilator assembly 200 may be configured to be steerable or fixed. The elongated sheath assembly 100 may be any type of dilator compatible with the elongated dilator assembly 200; the elongated sheath assembly 100 is configured to mate with the elongated dilator assembly 200, preferably at a proximal hub to prevent unwanted movement of the elongated sheath assembly 100.

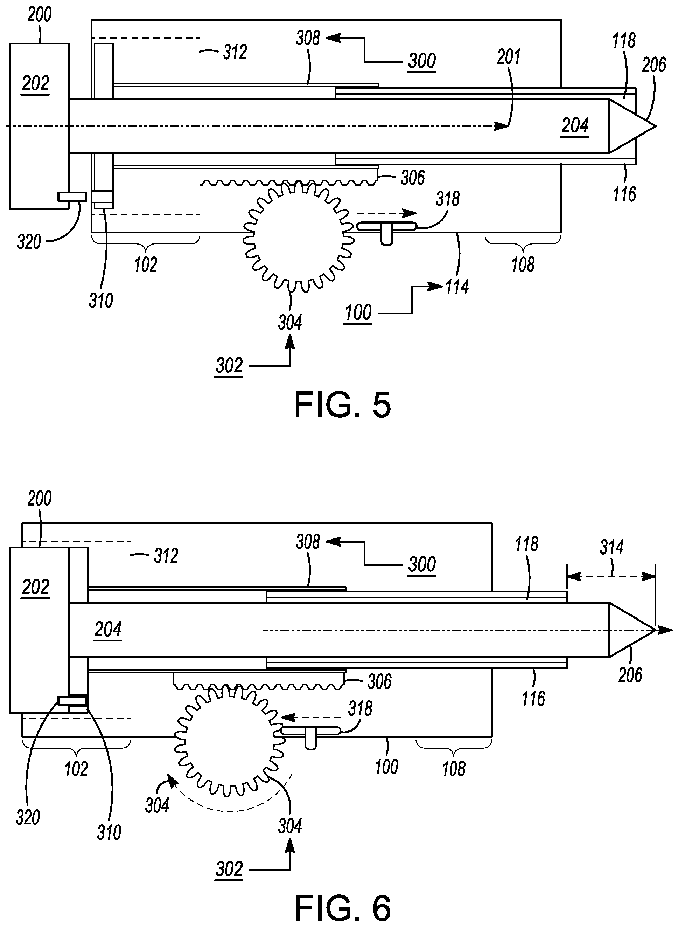

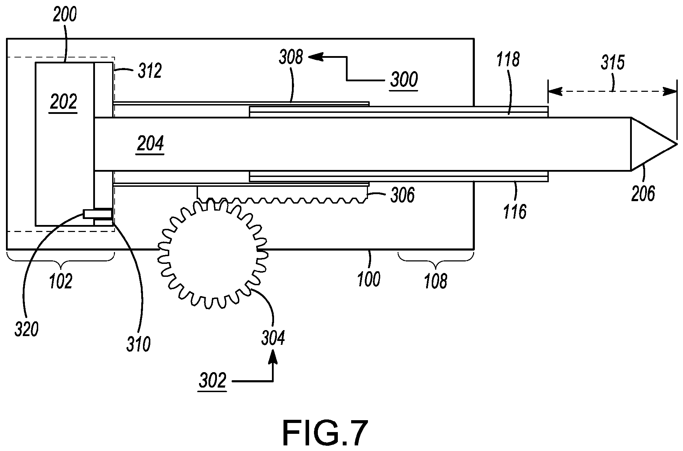

Referring to the embodiments as depicted in FIG. 3A, FIG. 3B and FIG. 4, the dilator-receiver mover 302 is configured (preferably) to selectively move the dilator-receiver device 300 from a first predetermined distance 314 (as depicted in FIG. 6) to a second predetermined distance 315 (as depicted in FIG. 7); this is done in such a way that the elongated dilator assembly 200 is also moveable from the first predetermined distance 314 (as depicted in FIG. 6) to the second predetermined distance 315. Preferably, this is done in such a way that the dilator-receiver mover 302 and the elongated dilator assembly 200 are moveable between the first predetermined distance 314 (as depicted in FIG. 6) and the second predetermined distance 315 (as depicted in FIG. 7); that is, while a portion (such as a dilator hub 202) of the elongated dilator assembly 200 and a portion (such as a sheath hub 310) of the elongated sheath assembly 100 remain in contact (in an abutment relationship or contact arrangement) with each other.

Referring to the embodiments as depicted in FIG. 3A, FIG. 3B and FIG. 4, in accordance with a preferred embodiment, the dilator-receiver mover 302 is configured (preferably) to be mounted (installed) to the elongated sheath assembly 100; this is done (preferably) in such a way that the dilator-receiver mover 302 selectively moves the dilator-receiver device 300 along a predetermined distance 314 (as depicted in FIG. 6). Referring back to FIG. 3A, FIG. 3B and FIG. 4, the dilator-receiver mover 302 may include a position-control device, a translation assembly, a gear assembly, and/or any equivalent thereof. For instance, the gear assembly (as depicted in FIG. 3A, FIG. 3B or FIG. 4) may include (and is not limited to) a synergistic combination of an input gear 304 and an output gear 306, etc. and/or any equivalent thereof. The input gear 304 is moveable (rotatable, reciprocatable) along a rotation direction 305. The output gear 306 is moveable along a reciprocation path 316 (preferably, a linear reciprocation path). The input gear 304 and the output gear 306 are configured to mesh with each other. The input gear 304 is operatively mounted to the elongated sheath assembly 100 (preferably mounted to the sheath-handle assembly 114 (or a housing) of the elongated sheath assembly 100). The output gear 306 is fixedly mounted to an outer surface of the moveable shaft 308 (or generally to the dilator-receiver device 300). Generally, the dilator-receiver mover 302 has a mover structure (such as the gear assembly, etc.) configured to be mounted (installed) to (a section or portion of) the elongated sheath assembly 100.

Referring to the embodiment as depicted in FIG. 3A, there is depicted a kit. The kit includes a first medical assembly 501. The first medical assembly 501 includes and is not limited to an elongated dilator assembly 200, a catheter, etc. and/or any equivalent thereof. The first medical assembly 501 and/or the elongated dilator assembly 200 is configured to be inserted into a confined space defined by a living body (a patient). The kit also includes a second medical assembly 502 configured to receive, at least in part, the first medical assembly 501. The second medical assembly 502 may include and is not limited to an elongated sheath assembly 100 and/or any equivalent thereof. The second medical assembly 502 is (preferably) configured to guide the insertion of the first medical assembly 501 into the confined space defined by the patient. The first medical assembly 501 and the second medical assembly 502 are (preferably) impermeable by a bodily fluid of the patient. The first medical assembly 501 and the second medical assembly 502 each includes (in accordance with a preferred embodiment) bio-compatible materials properties suitable for sufficient performance properties (dielectric strength, thermal performance, insulation and corrosion, water and heat resistance) for safe performance to comply with industrial and regulatory safety standards (or compatible for medical usage). Reference is made to the following publication for consideration in the selection of a suitable material: Plastics in Medical Devices: Properties, Requirements, and Applications; 2nd Edition; author: Vinny R. Sastri; hardcover ISBN: 9781455732012; published: 21 November 2013; publisher: Amsterdam [Pays-Bas]: Elsevier/William Andrew, [2014]. The kit also includes a receiver device 503 configured to be mounted, at least in part, to the second medical assembly 502. The receiver device 503 is configured to receive the first medical assembly 501. The receiver device 503 may include, and is not limited, to a dilator-receiver device 300 and anything equivalent thereof. The kit also includes a receiver mover 504 configured to be operatively connected to the receiver device 503; this is done in such a way that the receiver mover 504 selectively moves the receiver device 503 along a predetermined distance 314. The receiver mover 504 includes, and is not limited to, a dilator-receiver mover 302 and anything equivalent thereof.

Referring to the embodiments as depicted in FIG. 3A, FIG. 3B and FIG. 4, there is depicted an apparatus for use with (configured to be installed to) an elongated dilator assembly 200. The apparatus includes an elongated sheath assembly 100 configured to receive, at least in part, the elongated dilator assembly 200. A dilator-receiver device 300 is configured to be mounted, at least in part, to the elongated sheath assembly 100. A dilator-receiver mover 302 is operatively connected to the dilator-receiver device 300. Preferably, the dilator-receiver mover 302 is configured to selectively move the dilator-receiver device 300 along a predetermined distance 314.

Referring to the embodiments as depicted in FIG. 3A, FIG. 3B and FIG. 4, there is depicted an apparatus for (configured to be installed to) an elongated sheath assembly 100 configured to receive, at least in part, an elongated dilator assembly 200. The apparatus includes a dilator-receiver device 300, such as a moveable shaft 308 and/or any equivalent thereof. The dilator-receiver device 300 is mounted to (configured to be installed to, installed to, installed into, at least in part) the elongated sheath assembly 100. The dilator-receiver device 300 includes any suitable type of mount structure configured to be mounted, at least in part, to the elongated sheath assembly 100. A dilator-receiver mover 302 is configured to be operatively connected (directly or indirectly, coupled) to the dilator-receiver device 300. Preferably, the dilator-receiver mover 302 is configured to selectively move the dilator-receiver device 300 along a predetermined distance 314.

Referring to the embodiments as depicted in FIG. 3A, FIG. 3B and FIG. 4, the dilator-receiver mover 302 has a mounting structure configured to be mounted (installed) to the elongated sheath assembly 100.

Referring to the embodiments as depicted in FIG. 3A, FIG. 3B and FIG. 4, the dilator-receiver device 300 is configured to selectively receive and support, at least in part, the elongated dilator assembly 200; this is done, preferably, after the elongated sheath assembly 100, in use, receives, at least in part, the elongated dilator assembly 200.

Referring to the embodiments as depicted in FIG. 3A, FIG. 3B and FIG. 4, the dilator-receiver mover 302 is configured to operatively connect (or has a structure configured to connect or couple), either directly or indirectly, to the dilator-receiver device 300.

Referring to the embodiments as depicted in FIG. 3A, FIG. 3B and FIG. 4, the dilator-receiver mover 302 is configured to selectively move (translate, linearly translate), at least in part, the dilator-receiver device 300 and the elongated dilator assembly 200 after the elongated dilator assembly 200 is received, at least in part, by the dilator-receiver device 300 (as depicted in FIG. 6 and FIG. 7).

Referring to the embodiments as depicted in FIG. 3A, FIG. 3B and FIG. 4, the elongated sheath assembly 100 is configured to (slidably) receive, at least in part, the elongated dilator assembly 200 along, at least in part, a length of the elongated sheath assembly 100.

Referring to the embodiments as depicted in FIG. 3A, FIG. 3B and FIG. 4, the elongated sheath assembly 100 defines an exit portal 110. The elongated sheath assembly 100 includes a sheath hub 310 configured to be received, at least in part, into the hub cavity 312. The sheath hub 310 is also configured to be moveable, at least in part, along the hub cavity 312. The sheath hub 310 is also configured to abut, at least in part, a dilator hub 202 of the elongated dilator assembly 200; this is done, preferably, after the dilator hub 202 is positioned to abut, at least in part, the sheath hub 310 (preferably, after the sheath hub 310 is received, at least in part, into the hub cavity 312).

Referring to the embodiments as depicted in FIG. 3A, FIG. 3B and FIG. 4, the elongated sheath assembly 100 includes a first end section 102 (an entrance end) defining an entrance portal 104 leading to a hub cavity 312. A second end section 108 (an exit end) defines an exit portal 110. An elongated axis 103 extends axially between the first end section 102 and the second end section 108. A sheath hub 310 is configured to be received, at least in part, into the entrance portal 104 and the hub cavity 312 of the first end section 102. The sheath hub 310 is also configured to be moveable, at least in part, along the hub cavity 312 (along the elongated axis 103) after the sheath hub 310 is received, at least in part, in the hub cavity 312. The sheath hub 310 is also configured to abut, at least in part, a dilator hub 202 of the elongated dilator assembly 200; this is done, preferably, after the dilator hub 202 is positioned to abut, at least in part, the sheath hub 310 (preferably, after the sheath hub 310 is received, at least in part, into the hub cavity 312).

Referring to the embodiments as depicted in FIG. 3A, FIG. 3B and FIG. 4, the dilator-receiver mover 302 is positioned proximate to the sheath hub 310 of the elongated sheath assembly 100. The dilator-receiver mover 302 is configured to selectively move, at least in part, the sheath hub 310 and the dilator hub 202 between the first end section 102 and the second end section 108; this is done, preferably, after the sheath hub 310, in use, abuts (contacts), at least in part, the dilator hub 202.

Referring to the embodiments as depicted in FIG. 3A, FIG. 3B, the sheath assembly 100 also includes a sheath-handle assembly 114 (or a housing assembly) having a stationary shaft 116 defining a shaft lumen 118. The stationary shaft 116 fixedly extends from the sheath-handle assembly 114.

Referring to the embodiments as depicted in FIG. 3A, FIG. 3B and FIG. 4, the sheath hub 310 is configured to be movably supported by the sheath-handle assembly 114. The dilator-receiver device 300 is configured to selectively receive and support, at least in part, a dilator shaft 204 (elongated dilator shaft) and the dilator hub 202 of the elongated dilator assembly 200. The hub cavity 312 is in fluid communication with the shaft lumen 118 of the stationary shaft 116. The shaft lumen 118, of the stationary shaft 116, is configured to receive the dilator shaft 204 of the elongated dilator assembly 200. It will be appreciated that the dilator shaft 204 may include a polymer based material, and/or any suitable material(s). The dilator shaft 204 may include, for instance, SAE (Society of Automotive Engineering) Type 304 Stainless Steel. SAE Type 304 stainless steel contains both chromium (from between about 15% to about 20%) and nickel (from between about 2% to about 10.5%) metals as the main non-iron constituents. As an alternative, the dilator shaft 204 may include a shape-memory material configured to be manipulated and/or deformed followed by a return to the original shape that the shape-memory material was set in (prior to manipulation). Shape-memory materials (SMMs) are known and not further described in detail. Shape-memory materials are configured to recover their original shape from a significant and seemingly plastic deformation in response to a particular stimulus is applied to the shape-memory material. This is known as the shape memory effect (SME). Superelasticity (in alloys) may be observed once the shape-memory material is deformed under the presence (an application) of a stimulus force.

Referring to the embodiments as depicted in FIG. 3A, FIG. 3B and FIG. 4, the dilator-receiver device 300 includes a moveable shaft 308. The moveable shaft 308 is configured to be moveable within a longitudinal length of the elongated sheath assembly 100.

Referring to the embodiments as depicted in FIG. 3A, FIG. 3B and FIG. 4 (and FIG. 6 and FIG. 7), there is depicted a method of operating an elongated sheath assembly 100 configured to receive, at least in part, an elongated dilator assembly 200. The method includes selectively receiving and supporting, at least in part, the elongated dilator assembly 200 via a dilator-receiver device 300 mounted to, at least in part, the elongated sheath assembly 100. The method also includes selectively moving, at least in part, the dilator-receiver device 300 and the elongated dilator assembly 200 along a predetermined distance 314, via a dilator-receiver mover 302 operatively connected to the dilator-receiver device 300, after the elongated dilator assembly 200 is received, at least in part, by the dilator-receiver device 300. The method may include (in accordance with a preferred embodiment) having the dilator-receiver mover 302 mounted to the elongated sheath assembly 100.

Referring to the embodiment as depicted in FIG. 4 and FIG. 5, a lock device 320 (also called a latch device, etc.) may be provided. The lock device 320 may include a push-to-close-and-open latch (known to persons skilled in the art, and not depicted or described with further details). The lock device 320 is configured to selectively lock the sheath hub 310 and the dilator hub 202 to each other. The lock device 320 is also configured to selectively unlock the 310 and the dilator hub 202 from each other (for release purposes). The lock device 320 includes (preferably) a first lock portion 320A and a second lock portion 320B. The first lock portion 320A is mounted to the sheath hub 310 (that is, mounted to the surface of the sheath hub 310 that is exposed and facing outwardly (away) from the entrance portal 104). The second lock portion 320B is mounted to the dilator hub 202 (that is, to an outer surface of the dilator hub 202). Once the dilator hub 202 is positioned adjacent to (proximate to or abuts) the sheath hub 310, the first lock portion 320A and the second lock portion 320B are aligned with each other (as depicted in FIG. 5). The user may push the first lock portion 320A (of the dilator hub 202) into the second lock portion 320B (of the sheath hub 310); this action selectively latches the dilator hub 202 to the sheath hub 310 (with each other, as depicted in FIG. 6 and FIG. 7). Once the procedure is completed, the user may (when appropriate) reverse the locking action (latching process): the user may push to cause or urge the first lock portion 320A (of the dilator hub 202) temporarily into the second lock portion 320B (of the sheath hub 310); this action selectively unlatches (releases) the dilator hub 202 from the sheath hub 310); then, the elongated dilator assembly 200 may then be removed (entirely separated) away from the sheath assembly 100. It will be appreciated that the lock device 320 is (preferably) mounted in a centered position on the dilator hub 202 and the sheath hub 310, etc. (the mounting is depicted off-centered for purposes of clearly depicting the function and potential locations of the components of the lock device 320, for the sake of keeping the FIGS. relatively clean by placing the lock device 320 off to the side, off centered).

Referring to the embodiments as depicted in FIG. 5, the elongated dilator assembly 200 may be inserted into the sheath assembly 100 so that the dilator hub 202 may engage (contact, at least in part,) with the sheath hub 310. Protrusion of the dilator distal tip 206 may be minimized (if so desired, until the tip of the sheath assembly 100 is positioned for deployment of the elongated dilator assembly 200). The dilator assembly 200 is moveable along an insertion direction 201 aligned along, and within, the interior of the elongated sheath assembly 100 (such as the shaft lumen 118 and the moveable shaft 308). The shaft lumen 118 is configured to slidably receive the moveable shaft 308. The shaft lumen 118 is fixedly connected to the sheath-handle assembly 114. The moveable shaft 308 extends from the sheath hub 310. The sheath hub 310 is configured to be moveable along the hub cavity 312 (defined by the sheath-handle assembly 114).

Referring to the embodiments as depicted in FIG. 5, rotation of the input gear 304 causes the output gear 306 to become linearly translated, thereby linearly translating the moveable shaft 308 and the sheath hub 310. In use, rotation of the input gear 304 causes the output gear 306 to become linearly translated, thereby linearly translating the moveable shaft 308, the sheath hub 310 and the dilator hub 202 (after the dilator hub 202 is inserted into the sheath-handle assembly 114, and the dilator hub 202 is made to abut, or contact, the sheath hub 310).

Referring to the embodiments as depicted in FIG. 5, when the dilator-receiver mover 302 (preferably, the input gear 304) is activated (preferably, rotated), the sheath hub 310 is either moved forwards or backwards, effectively decreasing or increasing the total length of the elongated dilator assembly 200 that extends from the output section (such as the distal end section of the stationary shaft 116, of the elongated sheath assembly 100). In turn, the elongated dilator assembly 200 may either extend or retract from the distal tip of the stationary shaft 116 of the elongated sheath assembly 100. The moveable shaft 308 may slide (move) with the sheath hub 310 in response to activation of the dilator-receiver mover 302.

Referring to the embodiments as depicted in FIG. 5 and FIG. 6, the sheath hub 310 and the dilator hub 202 are configured to selectively lock together. A lock mechanism 318 (also called a latch mechanism) is configured to selectively securely lock (or latch as depicted in FIG. 6) the dilator-receiver mover 302 to the sheath-handle assembly 114 (or the elongated sheath assembly 100); this is done so that the dilator-receiver mover 302 is not movable, or not rotatable, relative to the elongated sheath assembly 100. The lock mechanism 318 is an optional feature. The lock mechanism 318 is configured to selectively unlock (unlatch as depicted in FIG. 5) the dilator-receiver mover 302 from the sheath-handle assembly 114 (or the elongated sheath assembly 100) so that the dilator-receiver mover 302 is movable, or rotatable, relative to the elongated sheath assembly 100. The lock mechanism 318 may include any suitable type of lock mechanism (and is not further described herein in any specific details since a person skilled in the art would know how to design and install the lock mechanism 318. The lock device 320 and the lock mechanism 318 may be deployed concurrently (together) or separately from each other (if so desired). In addition, the dilator-receiver mover 302 and the dilator-receiver device 300 provide an arrangement that improves precision adjustment of the protrusion length of the elongated dilator assembly 200 (extending from the end of the elongated sheath assembly 100) by the operator (preferably, after securely locking the sheath hub 310 and the dilator hub 202 together). In accordance with another embodiment, the lock device 320 is configured to selectively lock, and unlock, the sheath hub 310 to, and from, the dilator hub 202. The lock device 320 may include, for instance, a snap-fit connector, a press-fit connector, any type of locking device, etc., and/or any equivalent thereof.

Referring to the embodiment as depicted in FIG. 6, the sheath hub 310 (such as a port on the proximal end of the elongated sheath assembly 100) is configured to securely mate with the proximal end of the elongated dilator assembly 200. The position of the sheath hub 310 may be adjusted using the dilator-receiver mover 302 such that the elongated sheath assembly 100 may move (slide) further into the elongated sheath assembly 100 (preferably when mated with the dilator hub 202), or withdrawn from (from the interior of) the elongated sheath assembly 100. It will be appreciated that the lock device 320 (known and not described in detail) may be deployed (if required) between the dilator hub 202 and the sheath hub 310, and the lock device 320 may include any type of lock (e.g. snap fit, press fit, different shapes, materials, etc.), and/or any equivalent thereof. Preferably, the range of motion (of the dilator-receiver device 300 or the moveable shaft 308) is in the order of centimeters. The dilator-receiver mover 302 is configured to provide precise submillimeter resolution of linear movement of the dilator-receiver device 300. The dilator-receiver mover 302 may be located anywhere on the elongated sheath assembly 100 that is accessible by the operator.

Referring to the embodiment as depicted in FIG. 6, the dilator hub 202 of the elongated dilator assembly 200 makes contact with (engages) the sheath hub 310 of the elongated sheath assembly 100. The sheath hub 310 may be advanced forwardly (distally) via operation of the dilator-receiver mover 302, resulting in increased protrusion of the elongated dilator assembly 200 from the tip section of end section of the elongated sheath assembly 100.

Referring to the embodiment as depicted in FIG. 7, the dilator hub 202 of the elongated dilator assembly 200 continues to make contact with (engage) the sheath hub 310 of the elongated sheath assembly 100.

The sheath hub 310 is advanced to the most distal position or point, so that the protrusion of the elongated dilator assembly 200, from the distal tip section of the elongated sheath assembly 100, may be maximized (if, or when, desired during a procedure).

Referring to the embodiments as depicted in FIG. 6 and FIG. 7, the dilator-receiver mover 302 is configured (preferably) to selectively move the dilator-receiver device 300 from a first predetermined distance 314 (as depicted in FIG. 6) to a second predetermined distance 315 (as depicted in FIG. 7); this is done in such a way that the elongated dilator assembly 200 is also moveable from the first predetermined distance 314 (as depicted in FIG. 6) to the second predetermined distance 315. Preferably, this is done in such a way that the dilator-receiver mover 302 and the elongated dilator assembly 200 are moveable between the first predetermined distance 314 (as depicted in FIG. 6) and the second predetermined distance 315 (as depicted in FIG. 7); that is, while a portion (such as, a dilator hub 202) of the elongated dilator assembly 200 and a portion (such as a sheath hub 310) of the elongated sheath assembly 100 remain in contact (in an abutment relationship) with each other. Advantageously, the elongated sheath assembly 100 may be configured to avoid swapping out the elongated dilator assembly 200 (during a procedure, if desired), thereby helping the surgeon avoid wasting valuable or critical surgical time. Advantageously, the elongated sheath assembly 100 may provide a workflow that starts with a long reach and tenting (over-tenting) the septum 902 (depicted in FIG. 2B), then withdrawing the dilator assembly 200 to an appropriate degree (or amount) of tent formed in a portion of the septum 902; it will be appreciated that this methodology may be more intuitive than changing the curve of the dilator assembly 200. Advantageously, the additional length of the elongated dilator assembly 200, which may become moved and exposed during a procedure (that is, extended out from the end of the elongated sheath assembly 100), may vary in an amount, or a length (a reasonable length), of an exposed portion of the dilator distal tip 206 (which extends from the end of the elongated sheath assembly 100). This may be done, if desired, without having to swap out the elongated dilator assembly 200 during the procedure (thereby saving valuable or critical surgical time). For the case where the elongated dilator assembly 200 does not need to be swapped out for length issues, the elongated dilator assembly 200 may be withdrawn to adjust the curve of the elongated dilator assembly 200. The dilator assembly 200 may be selectively extended, or moved, further out from (the output of) the sheath assembly 100, while the elongated dilator assembly 200 remains mated (securely mated or in contact with) to the sheath assembly 100 during a procedure, so that the elongated dilator assembly 200 may become selectively exposed from the sheath assembly 100 (during the procedure). There is a range of positions (range of hub positions) spanning between the first predetermined distance 314 (as depicted in FIG. 6) and the second predetermined distance 315, in which the dilator-receiver device 300 (the moveable shaft 308) may be moveable (reciprocated along a reciprocation path 316, as depicted in FIG. 3A, FIG. 3B). The dilator-receiver device 300 and the dilator-receiver mover 302 advantageously provide physicians additional confidence and/or control during transseptal puncture (a procedure) such that the force to be applied to the septum has an appropriate level of impact to the tissue of the patient.

The following is offered as further description of the embodiments, in which any one or more of any technical feature (described in the detailed description, the summary and the claims) may be combinable with any other one or more of any technical feature (described in the detailed description, the summary and the claims). It is understood that each claim in the claims section is an open ended claim unless stated otherwise. Unless otherwise specified, relational terms used in these specifications should be construed to include certain tolerances that the person skilled in the art would recognize as providing equivalent functionality. By way of example, the term perpendicular is not necessarily limited to 90.0 degrees, and may include a variation thereof that the person skilled in the art would recognize as providing equivalent functionality for the purposes described for the relevant member or element. Terms such as "about" and "substantially", in the context of configuration, relate generally to disposition, location, or configuration that are either exact or sufficiently close to the location, disposition, or configuration of the relevant element to preserve operability of the element within the disclosure which does not materially modify the disclosure. Similarly, unless specifically made clear from its context, numerical values should be construed to include certain tolerances that the person skilled in the art would recognize as having negligible importance as they do not materially change the operability of the disclosure. It will be appreciated that the description and/or drawings identify and describe embodiments of the apparatus (either explicitly or inherently). The apparatus may include any suitable combination and/or permutation of the technical features as identified in the detailed description, as may be required and/or desired to suit a particular technical purpose and/or technical function. It will be appreciated that, where possible and suitable, any one or more of the technical features of the apparatus may be combined with any other one or more of the technical features of the apparatus (in any combination and/or permutation). It will be appreciated that persons skilled in the art would know that the technical features of each embodiment may be deployed (where possible) in other embodiments even if not expressly stated as such above. It will be appreciated that persons skilled in the art would know that other options may be possible for the configuration of the components of the apparatus to adjust to manufacturing requirements and still remain within the scope as described in at least one or more of the claims. This written description provides embodiments, including the best mode, and also enables the person skilled in the art to make and use the embodiments. The patentable scope may be defined by the claims. The written description and/or drawings may help to understand the scope of the claims. It is believed that all the crucial aspects of the disclosed subject matter have been provided in this document. It is understood, for this document, that the word "includes" is equivalent to the word "comprising" in that both words are used to signify an open-ended listing of assemblies, components, parts, etc. The term "comprising", which is synonymous with the terms "including," "containing," or "characterized by," is inclusive or open-ended and does not exclude additional, unrecited elements or method steps. Comprising (comprised of) is an "open" phrase and allows coverage of technologies that employ additional, unrecited elements. When used in a claim, the word "comprising" is the transitory verb (transitional term) that separates the preamble of the claim from the technical features of the disclosure. The foregoing has outlined the non-limiting embodiments (examples). The description is made for particular non-limiting embodiments (examples). It is understood that the non-limiting embodiments are merely illustrative as examples.

## Claims

1. An apparatus for an elongated sheath assembly (100) configured to receive, at least in part, an elongated dilator assembly (200), and the apparatus comprising:
a dilator-receiver device (300) mounted, at least in part, to the elongated sheath assembly (100);
**characterized by**
a dilator-receiver mover (302) configured to be operatively connected to the dilator- receiver device, and the dilator-receiver mover (302) also configured to selectively move the dilator-receiver device (300) along a predetermined distance; and optionally
the dilator-receiver mover (302) is mounted to the elongated sheath assembly (100).

2. The apparatus of claim 1, wherein:
the dilator-receiver device (300) is configured to selectively receive and support, at least in part, the elongated dilator assembly (200) after the elongated sheath assembly (100), in use, receives, at least in part, the elongated dilator assembly (200).

3. The apparatus of claim 1, wherein:
the dilator-receiver mover (302) is configured to operatively connect to the dilator- receiver device; and/or
the dilator-receiver mover (302) is configured to selectively move, at least in part, the dilator-receiver device (300) and the elongated dilator assembly (200) after the elongated dilator assembly (200) is received, at least in part, by the dilator-receiver device (300).

4. The apparatus of claim 1, wherein:
the elongated sheath assembly (100) is configured to receive, at least in part, the elongated dilator assembly (200) along, at least in part, a length of the elongated sheath assembly (100).

5. The apparatus of claim 1, wherein:
the elongated sheath assembly (100) includes:
a first end section (102, 402) defining an entrance portal (104, 404) leading to a hub cavity (312); and a second end section (108, 408) defining an exit portal (110, 409); and
a sheath hub (310, 410) configured to be received, at least in part, into the entrance portal (104, 404) and the hub cavity (312) of the first end section (102, 402); and
the sheath hub (310, 410) also configured to be moveable, at least in part, along the hub cavity (312) after the sheath hub (310, 410) is received, at least in part, in the hub cavity (312); and
the sheath hub (310, 410) also configured to abut, at least in part, a dilator hub (202) of the elongated dilator assembly (200) after the dilator hub (202) is positioned to abut, at least in part, the sheath hub (310, 410);
and, optionally, the elongated sheath assembly (100) further includes a lock device (320) is installed to the sheath hub (310, 410) and the dilator hub (202); and
the lock device (320) is configured to selectively lock, and unlock, the sheath hub (310, 410) to, and from, the dilator hub (202).

6. The apparatus of claim 5, wherein:
the dilator-receiver mover (302) is positioned proximate to the sheath hub (310, 410) of the elongated sheath assembly (100); and
the dilator-receiver mover (302) is configured to selectively move, at least in part, the sheath hub (310, 410) and the dilator hub (202) between the first end section (102, 402) and the second end section (108, 408) after the sheath hub (310, 410), in use, abuts, at least in part, the dilator hub (202).

7. The apparatus of claim 6, wherein:
the elongated sheath assembly (100) also includes:
a sheath-handle assembly (414) having a stationary shaft (116, 416) defining a shaft lumen (118, 418); and the stationary shaft (116, 416) fixedly extending from the sheath-handle assembly (414).

8. The apparatus of claim 7, wherein:
the sheath hub (310, 410) is configured to be movably supported by the sheath-handle assembly (414); and/or
the dilator-receiver device (300) is configured to selectively receive and support, at least in part, a dilator shaft (204) and the dilator hub (202) of the elongated dilator assembly (200).

9. The apparatus of claim 8, wherein:
the hub cavity (312) is in fluid communication with the shaft lumen (118, 418) of the stationary shaft (116, 416) and/or
the shaft lumen (118, 418), of the stationary shaft (116, 416), is configured to receive a dilator shaft (204) of the elongated dilator assembly (200).

10. The apparatus of claim 1, wherein:
the dilator-receiver device (300) includes a moveable shaft; and/or
the dilator-receiver mover (302) is configured to selectively move the dilator-receiver device (300) from a first predetermined distance to a second predetermined distance in such a way that the elongated dilator assembly (200) is also moveable from the first predetermined distance to the second predetermined distance.

11. The apparatus of claim 1, wherein:
the dilator-receiver mover (302) includes: a gear assembly including:
an input gear (304); and
an output gear (306) configured to mesh with the input gear (304); and
wherein:
the input gear (304) is moveable along a rotation direction; and the output gear (306) is moveable along a reciprocation path (316); and
the input gear (304) is operatively mounted to a sheath-handle assembly (414) of the elongated sheath assembly (100); and
the output gear (306) is fixedly mounted to the dilator-receiver device (300).

12. The apparatus of claim 1, further comprising:
a lock mechanism configured to selectively lock the dilator-receiver mover (302) to the elongated sheath assembly (100); and
the lock mechanism also configured to selectively unlock the dilator-receiver mover (302) from the elongated sheath assembly (100).

13. A kit, comprising:
an elongated dilator assembly (200); and
an elongated sheath assembly (100) configured to receive, at least in part, the elongated dilator assembly (200); and
an apparatus according to any one of the preceding claims.

## Patentansprüche

1. Vorrichtung für eine längliche Hüllenanordnung (100), die eingerichtet ist, eine längliche Dilatatoranordnung (200) zumindest teilweise aufzunehmen, wobei die Vorrichtung umfasst:
eine Dilatator-Aufnahmevorrichtung (300), die zumindest teilweise an der länglichen Hüllenanordnung (100) angebracht ist;
**gekennzeichnet durch**
eine Dilatator-Aufnahme-Bewegungseinheit (302), die eingerichtet ist, funktionsmäßig mit der Dilatator-Aufnahmevorrichtung verbunden zu sein, und wobei die Dilatator-Aufnahme-Bewegungseinheit (302) auch eingerichtet ist, die Dilatator-Aufnahme-Bewegungseinheit (300) entlang einem vorgegeben Abstand selektiv zu bewegen; und wobei optional
die Dilatator-Aufnahme-Bewegungseinheit (302) an der länglichen Hüllenanordnung (100) angebracht ist.

2. Vorrichtung nach Anspruch 1, wobei:
die Dilatator-Aufnahmevorrichtung (300) eingerichtet ist, die längliche Dilatatoranordnung (200) selektiv aufzunehmen und zumindest teilweise zu stützen, nachdem die längliche Hüllenanordnung (100) im Gebrauch die längliche Dilatatoranordnung (200) zumindest teilweise aufnimmt.

3. Vorrichtung nach Anspruch 1, wobei:
die Dilatator-Aufnahme-Bewegungseinheit (302) eingerichtet ist, funktionsmäßig mit der Dilatator-Aufnahmevorrichtung verbunden zu sein; und/oder
die Dilatator-Aufnahme-Bewegungseinheit (302) eingerichtet ist, die Dilatator-Aufnahmevorrichtung (300) und die längliche Dilatatoranordnung (200) zumindest teilweise selektiv zu bewegen, nachdem die längliche Dilatatoranordnung (200) zumindest teilweise von der Dilatator-Aufnahmevorrichtung (300) aufgenommen wird.

4. Vorrichtung nach Anspruch 1, wobei:
die längliche Hüllenanordnung (100) eingerichtet ist, zumindest teilweise die längliche Dilatatoranordnung (200) entlang zumindest eines Teils der Länge der länglichen Hüllenanordnung (100) aufzunehmen.

5. Vorrichtung nach Anspruch 1, wobei:
die längliche Hüllenanordnung (100) umfasst:
einen ersten Endabschnitt (102, 402), der ein Eingangsportal (104, 404) definiert, das zu einem Nabenhohlraum (312) führt; und einen zweiten Endabschnitt (108, 408), der ein Ausgangsportal (110, 409) definiert; und
eine Hüllennabe (310, 410), die eingerichtet ist, zumindest teilweise in dem Eingangsportal (104, 404) und dem Nabenhohlraum (312) des ersten Endabschnitts (102, 402) aufgenommen zu werden; und
wobei die Hüllennabe (310, 410) auch eingerichtet ist, nachdem die Hüllennabe (310, 410) zumindest teilweise in dem Nabenhohlraum (312) aufgenommen wird, entlang des Nabenhohlraums (312) zumindest teilweise bewegbar zu sein; und
wobei die Hüllennabe (310, 410) auch eingerichtet ist, nachdem die Dilatatornabe (202) so positioniert ist, dass sie zumindest teilweise an der Hüllennabe (310, 410) anliegt, an einer Dilatatornabe (202) der länglichen Dilatatoranordnung (200) zumindest teilweise anzuliegen;
und die längliche Hüllenanordnung (100) ferner eine Verriegelungsvorrichtung (320) optional umfasst, die an der Hüllennabe (310, 410) und der Dilatatornabe (202) installiert ist; und
die Verriegelungsvorrichtung (320) eingerichtet ist, die Hüllennabe (310, 410) selektiv mit der Dilatatornabe (202) zu verriegeln und von ihr zu entriegeln.

6. Vorrichtung nach Anspruch 5, wobei:
die Dilatator-Aufnahme-Bewegungseinheit (302) in der Nähe der Hüllennabe (310, 410) der länglichen Hüllenanordnung (100) angeordnet ist; und
die Dilatator-Aufnahme-Bewegungseinheit (302) eingerichtet ist, die Hüllennabe (310, 410) und die Dilatatornabe (202) selektiv zwischen dem ersten Endabschnitt (102, 402) und dem zweiten Endabschnitt (108, 408) zumindest teilweise zu bewegen, nachdem die Hüllennabe (310, 410) im Gebrauch zumindest teilweise an der Dilatatornabe (202) anliegt.

7. Vorrichtung nach Anspruch 6, wobei:
die längliche Hüllenanordnung (100) auch umfasst:
eine Hüllengriffanordnung (414) mit einem unbeweglichen Schaft (116, 416), der ein Schaftlumen (118, 418) definiert; und wobei sich der unbewegliche Schaft (116, 416) fest von der Hüllengriffanordnung (414) erstreckt.

8. Vorrichtung nach Anspruch 7, wobei:
die Hüllennabe (310, 410) eingerichtet ist, um von der Hüllengriffanordnung (414) beweglich gestützt zu werden; und/oder
die Dilatator-Aufnahmevorrichtung (300) eingerichtet ist, selektiv einen Dilatatorschaft (204) und die Dilatatornabe (202) der länglichen Dilatatoranordnung (200) zumindest teilweise aufzunehmen und zu stützen.

9. Vorrichtung nach Anspruch 8, wobei:
der Nabenhohlraum (312) in Fluidverbindung mit dem Schaftlumen (118, 418) des unbeweglichen Schafts (116, 416) steht und/oder
das Schaftlumen (118, 418) des unbeweglichen Schafts (116, 416) eingerichtet ist, einen Dilatatorschaft (204) der länglichen Dilatatoranordnung (200) aufzunehmen.

10. Vorrichtung nach Anspruch 1, wobei:
die Dilatator-Aufnahmevorrichtung (300) einen beweglichen Schaft umfasst; und/oder
die Dilatator-Aufnahme-Bewegungseinheit (302) eingerichtet ist, selektiv die Dilatator-Aufnahmevorrichtung (300) von einem ersten vorgegebenen Abstand zu einem zweiten vorgegebenen Abstand zu bewegen, so dass die längliche Dilatatoranordnung (200) auch von dem ersten vorgegebenen Abstand zu dem zweiten vorgegebenen Abstand bewegbar ist.

11. Vorrichtung nach Anspruch 1, wobei:
die Dilatator-Aufnahme-Bewegungseinheit (302) umfasst: eine Getriebeanordnung umfassend:
ein Eingangszahnrad (304); und
ein Ausgangszahnrad (306), eingerichtet, um in das Eingangszahnrad (304) einzugreifen; und
wobei:
das Eingangszahnrad (304) entlang einer Drehrichtung bewegbar ist; und
das Ausgangszahnrad (306) entlang eines Reziprozierwegs (316) bewegbar ist; und
das Eingangszahnrad (304) funktionsmäßig an einer Hüllengriffanordnung (414) der länglichen Hüllenanordnung (100) angebracht ist; und
das Ausgangszahnrad (306) fest mit der Dilatator-Aufnahmevorrichtung (300) verbunden ist.

12. Vorrichtung nach Anspruch 1, ferner umfassend:
einen Sperrmechanismus, der eingerichtet ist, die Dilatator-Aufnahme-Bewegungseinheit (302) selektiv an der länglichen Hülsenanordnung (100) zu verriegeln; und
wobei der Sperrmechanismus auch eingerichtet ist, die Dilatator-Aufnahme-Bewegungseinheit (302) selektiv von der länglichen Hüllenanordnung (100) zu entriegeln.

13. Kit, umfassend:
eine längliche Dilatatoranordnung (200); und
eine längliche Hüllenanordnung (100), die eingerichtet ist, die längliche Dilatatoranordnung (200) zumindest teilweise aufzunehmen; und
eine Vorrichtung nach einem der vorhergehenden Ansprüche.

## Revendications

1. Appareil pour un ensemble de gaine allongée (100) configuré pour recevoir, au moins en partie, un ensemble de dilatateur allongé (200), et l'appareil comprenant :
un dispositif de récepteur de dilatateur (300) monté, au moins en partie, sur l'ensemble de gaine allongée (100) ;
**caractérisé par** :
un dispositif de déplacement de récepteur de dilatateur (302) configuré pour être connecté de manière opérationnelle au dispositif de récepteur de dilatateur, et le dispositif de déplacement de récepteur de dilatateur (302) étant également configuré pour déplacer de façon sélective le dispositif de récepteur de dilatateur (300) sur une distance prédéterminée ; et en option :
le dispositif de déplacement de récepteur de dilatateur (302) est monté sur l'ensemble de gaine allongée (100).

2. Appareil selon la revendication 1, dans lequel :
le dispositif de récepteur de dilatateur (300) est configuré pour recevoir et supporter de façon sélective, au moins en partie, l'ensemble de dilatateur allongé (200) après que l'ensemble de gaine allongée (100), en utilisation, a reçu, au moins en partie, l'ensemble de dilatateur allongé (200).

3. Appareil selon la revendication 1, dans lequel :
le dispositif de déplacement de récepteur de dilatateur (302) est configuré pour être connecté de manière opérationnelle au dispositif de récepteur de dilatateur ; et/ou
le dispositif de déplacement de récepteur de dilatateur (302) est configuré pour déplacer de façon sélective, au moins en partie, le dispositif de récepteur de dilatateur (300) et l'ensemble de dilatateur allongé (200) après que l'ensemble de dilatateur allongé (200) a été reçu, au moins en partie, par le dispositif de récepteur de dilatateur (300).

4. Appareil selon la revendication 1, dans lequel :
l'ensemble de gaine allongée (100) est configuré pour recevoir, au moins en partie, l'ensemble de dilatateur allongé (200) sur, au moins en partie, une longueur de l'ensemble de gaine allongée (100).

5. Appareil selon la revendication 1, dans lequel :
l'ensemble de gaine allongée (100) inclut :
une première section d'extrémité (102, 402) qui définit un portail d'entrée (104, 404) qui débouche sur une cavité de moyeu (312) ; et une seconde section d'extrémité (108, 408) qui définit un portail de sortie (110, 409) ; et
un moyeu de gaine (310, 410) configuré pour être reçu, au moins en partie, à l'intérieur du portail d'entrée (104, 404) et de la cavité de moyeu (312) de la première section d'extrémité (102, 402) ; et
le moyeu de gaine (310, 410) étant également configuré pour pouvoir être déplacé, au moins en partie, le long de la cavité de moyeu (312) après que le moyeu de gaine (310, 410) a été reçu, au moins en partie, dans la cavité de moyeu (312) ; et
le moyeu de gaine (310, 410) étant également configuré pour venir en butée, au moins en partie, contre un moyeu de dilatateur (202) de l'ensemble de dilatateur allongé (200) après que le moyeu de dilatateur (202) a été positionné pour venir en butée, au moins en partie, contre le moyeu de gaine (310, 410) ;
et en option, l'ensemble de gaine allongée (100) inclut en outre un dispositif de blocage (320) qui est installé sur le moyeu de gaine (310, 410) et sur le moyeu de dilatateur (202) ; et
le dispositif de blocage (320) est configuré pour, de façon sélective, bloquer le moyeu de gaine (310, 410) sur le moyeu de dilatateur (202) et pour l'en débloquer.

6. Appareil selon la revendication 5, dans lequel :
le dispositif de déplacement de récepteur de dilatateur (302) est positionné à proximité du moyeu de gaine (310, 410) de l'ensemble de gaine allongée (100) ; et
le dispositif de déplacement de récepteur de dilatateur (302) est configuré pour déplacer de façon sélective, au moins en partie, le moyeu de gaine (310, 410) et le moyeu de dilatateur (202) entre la première section d'extrémité (102, 402) et la seconde section d'extrémité (108, 408) après que le moyeu de gaine (310, 410), en utilisation, est venu en butée, au moins en partie, contre le moyeu de dilatateur (202).

7. Appareil selon la revendication 6, dans lequel :
l'ensemble de gaine allongée (100) inclut également :
un ensemble de poignée de gaine (414) comportant un arbre stationnaire (116, 416) définissant une lumière d'arbre (118, 418) ; et l'arbre stationnaire (116, 416) étant étendu de manière fixe depuis l'ensemble de poignée de gaine (414).

8. Appareil selon la revendication 7, dans lequel :
le moyeu de gaine (310, 410) est configuré pour être supporté de façon amovible et mobile par l'ensemble de poigné de gaine (414) ; et/ou
le dispositif de récepteur de dilatateur (300) est configuré pour recevoir et supporter de façon sélective, au moins en partie, un arbre de dilatateur (204) et le moyeu de dilatateur (202) de l'ensemble de dilatateur allongé (200).

9. Appareil selon la revendication 8, dans lequel :
la cavité de moyeu (312) est en communication fluidique avec la lumière d'arbre (118, 418) de l'arbre stationnaire (116, 416) ; et/ou
la lumière d'arbre (118, 418) de l'arbre stationnaire (116, 416) est configurée pour recevoir un arbre de dilatateur (204) de l'ensemble de dilatateur allongé (200).

10. Appareil selon la revendication 1, dans lequel :
le dispositif de récepteur de dilatateur (300) inclut un arbre mobile ; et/ou
le dispositif de déplacement de récepteur de dilatateur (302) est configuré pour déplacer de façon sélective le dispositif de récepteur de dilatateur (300) depuis une première distance prédéterminée jusqu'à une seconde distance prédéterminée de telle sorte que l'ensemble de dilatateur allongé (200) puisse également être déplacé depuis la première distance prédéterminée jusqu'à la seconde distance prédéterminée.

11. Appareil selon la revendication 1, dans lequel :
le dispositif de déplacement de récepteur de dilatateur (302) inclut : un ensemble de roues dentées incluant :
une roue dentée d'entrée (304) ; et
une roue dentée de sortie (306) configurée pour être engrenée avec la roue dentée d'entrée (304) ; et
dans lequel :
la roue dentée d'entrée (304) peut être déplacée suivant une direction de rotation ;
et la roue dentée de sortie (306) peut être déplacée suivant une trajectoire de déplacement en va-et-vient (316) ; et
la roue dentée d'entrée (304) est montée de manière opérationnelle sur un ensemble de poignée de gaine (414) de l'ensemble de gaine allongée (100) ; et
la roue dentée de sortie (306) est montée de manière fixe sur le dispositif de récepteur de dilatateur (300).

12. Appareil selon la revendication 1, comprenant en outre :
un mécanisme de blocage configuré pour bloquer de façon sélective le dispositif de déplacement de récepteur de dilatateur (302) sur l'ensemble de gaine allongée (100) ; et
le mécanisme de blocage étant également configuré pour débloquer de façon sélective le dispositif de déplacement de récepteur de dilatateur (302) par rapport à l'ensemble de gaine allongée (100).

13. Kit, comprenant :
un ensemble de dilatateur allongé (200) ; et
un ensemble de gaine allongée (100) configuré pour recevoir, au moins en partie, l'ensemble de dilatateur allongé (200) ; et
un appareil selon l'une quelconque des revendications précédentes.
